(19) Europäisches Patentamt

European Patent Office

Office européen des brevets

(11) Publication number: **0 295 912 B1**

(12) **EUROPEAN PATENT SPECIFICATION**

(45) Date of publication of patent specification: **22.04.92** (51) Int. Cl.⁵: **A61F 2/34**

(21) Application number: **88305503.0**

(22) Date of filing: **16.06.88**

(54) **Acetabular cup.**

(30) Priority: **17.06.87 GB 8714141**

(43) Date of publication of application:
**21.12.88 Bulletin 88/51**

(45) Publication of the grant of the patent:
**22.04.92 Bulletin 92/17**

(84) Designated Contracting States:
**DE ES GB IT**

(56) References cited:
**EP-A- 0 137 664**
**EP-A- 0 190 093**
**EP-A- 0 214 885**
**EP-A- 0 242 719**

(73) Proprietor: **JOHNSON & JOHNSON ORTHO-PAEDICS INC.**
**501 George Street**
**New Brunswick New Jersey 08903(US)**

(72) Inventor: **Ritchie, Allan**
**11 Anderwood Drive Sway**
**Lymington Hampshire, SO41 6AW(GB)**
Inventor: **Tucker, Keith**
**77 Newmarket Road**
**Norwich Norfolk(GB)**

(74) Representative: **Fisher, Adrian John et al**
**CARPMAELS & RANSFORD 43 Bloomsbury Square**
**London WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to artificial hip joints, and more particularly to the cups which constitute the acetabular component of such artificial joints.

Acetabular cups are widely used for patients whose natural acetabulum no longer provides an appropriate bearing surface for the head of the femur. Countless different designs have been proposed in the literature, and indeed a large number of such designs are available commercially. For cemented hips, acetabular cups are generally in the form of a hollow hemisphere of polyethylene, into which the femoral head is received in the assembled joint. An alternative form of cup, which is more usual in cementless hips, comprising a hemispherical metal shell inside which a polyethylene lining is fixed.

Four main methods are currently used for securing an acetabular cup to a patient's acetabulum, these methods being used either singly or in various combinations. These methods are:
1. the use of a bone cement,
2. the use of bone screws,
3. the provision of a screw thread around the outer surface of the metal shell, so that the cup can be screwed directly into a suitably prepared acetabulum, and
4. the use of a porous coating on the outer surface of the cup to promote growth of bone into minute interstices in the coating.

Whichever method of fixation is adopted, it is desirable for the natural acetabulum to be as intact as possible. In many cases, however, the natural acetabulum is partly damaged or deficient. Rheumatoid arthritis and osteoarthritis, for example, are conditions which quite commonly lead to breakdown of the acetabular wall, whether by mechanical damage or by stress protection osteolysis. In such cases it is usual to make up the deficiency in the acetabular wall by means of a bone graft prior to fixation of an acetabular cup. In some cases, the graft is achieved by attaching solid pieces of bone (such as from the femoral head or the iliac crest) in the position of the defect with bone screws. The graft is then shaped using bone cutting tools to receive the acetabular cup. In an alternative procedure, the graft material is pulped into a paste-like consistency, and then packed into the area of the defect. In both techniques, there is a degree of difficulty in holding the graft material in position, and this applies in particular when pulped graft material is used.

EP-A-242719, which forms part of the state of the art in respect of DE, GB and IT by virtue of Article 54(3) EPC, discloses an acetabular cup having a plurality of support plates extending therefrom. These support plates appear to be suitable for supporting bone grafts. The cup disclosed is manufactured from plastics material.

According to the present invention there is provided an acetabular cup having a graft support plate extending therefrom to support a bone graft in the vicinity of the cup. In particular, there is provided an acetabular cup according to claim 1 hereof.

The graft support plate may be any of a wide range of shapes and sizes. In order to give the maximum possible freedom to the surgeon, the graft support plate will not usually be supplied attached to the cup, but will be supplied separately for subsequent attachment to the cup by the surgeon. Preferably, a range of graft support plates will be supplied, to cater for differing degrees of deficiency in the acetabulum. Alternatively, or in addition, the graft support plate may be made of a material which can be cut to suit the patient's needs, and which is sufficiently deformable to allow the surgeon to bend it to the appropriate shape. If, as is preferred, the graft support plate is formed from a titanium alloy, these properties can be obtained by the use of a plate which is from 0.05 to 1.00 mm thick, and preferably from 0.1 to 0.6 mm thick. It is particularly preferred that the plate be from 0.15 mm to 0.4 mm thick, e.g. 0.25 mm.

The graft support plate can conveniently be of generally rectangular shape, preferably with rounded corners to avoid damage to adjacent tissue when in use. A typical size for such a plate would be from 2.5 to 10 cm in length and from 1.5 to 6 cm in width. Preferably, the length of a generally rectangular plate will be from 4 to 8 cm and more preferably from 5 to 7.5 cm, e.g. 6.3 cm. The width of such a plate is preferably from 2 to 5 cm, and more preferably from 3 to 4.5 cm, e.g. 3.6 cm.

The graft support plate may alternatively be in the shape of an ellipse, a segment of a circle or an ellipse, or a crescent shape. Other shapes will also occur to those skilled in the art.

In order to facilitate attachment of the graft support plate to the pelvis (or to the graft when the latter comprises pieces of bone of substantial size), the plate may be provided with a plurality of apertures to receive bone screws. From 5 to 50 of such apertures will conveniently be provided, and preferably from 10 to 20. Each aperture may, for example, be from 2 to 6 mm in diameter, and preferably from 3 to 5 mm, e.g. 4 mm.

Preferably, the cup is in the form of a hollow generally hemispherical body, and the graft support plate is attached thereto adjacent the annular end surface of the body.

In a particularly preferred embodiment, the cup comprises an outer body in the form of a generally hemispherical shell, and a hemispherical lining which (in use of the cup) is received therein, the graft support plate extending from an annular member sandwiched between the annular end surface of the outer shell and an annular rim extending around the periphery of the hemispherical lining. This embodiment has the advantage that it is very easily assembled by the surgeon, but it is desirable to provide means (other than the friction between the annular member attached to the graft support plate and the annular end surface of the outer shell) to prevent rotation of the graft support plate relative to the cup. These means preferably take the form of a small spigot provided on the annular end surface of the shell, and a corresponding aperture provided in the annular member.

The lining may be retained in the outer shell by any of a variety of means. It is particularly preferred, however, that the lining be secured in the outer shell by means of a spring clip which in the assembled cup is received partly in an annular groove in the internal spherical surface of the outer shell and partly in a corresponding annular groove in the outer spherical surface of the lining.

The spring clip is preferably in the form of a part-circle of suitably resilient wire, having end portions which are bent out of the plane of the circle and which preferably lie generally perpendicular thereto. Such end portions not only serve as a convenient means of manipulating the clip during assembly of the cup, but they also ensure that in the assembled cup the clip is not completely buried in the groove in the outer shell. This latter condition must of course be avoided, otherwise the clip will not form the desired connection between the lining and the outer shell.

If, as in the preferred embodiment, the lining is provided with a peripheral rim, such rim should have a slot therein to allow the upturned end portions of the spring clip to be accommodated. The slot will conveniently be of arcuate shape.

An alternative method of ensuring that the spring clip is not completely received within the groove in the outer shell is to arrange that the groove is shallower than the diameter of the wire from which the clip is formed. For example, the groove in the shell may have a depth which is from 25% to 75% of the diameter of the wire.

The outer shell is preferably formed from a metal such as titanium or a titanium alloy. Stainless steel and cobalt chrome alloys are also suitable, the latter preferably being in accordance with British Standard No. 3531. The graft support plate may be made from the same material as the outer shell. Other materials may be used, however, provided that they are biocompatible and sufficiently rigid for the purpose, and further provided that they do not cause corrosion reactions with the material of the outer shell. For the latter reason, the use of a stainless steel cup with a titanium or cobalt chrome alloy graft support plate (or vice versa) should be avoided if the two parts contact each other. If desired, the graft support plate may be of a plastics material such as an acetal resin or an ultra high molecular weight polyethylene. Particularly suitable acetal resins are available under the Trade Mark DELRIN. The lining is preferably formed from a plastics material, such as high density polyethylene.

The outer surface of the outer shell may optionally be configured to promote ingrowth of bone. If such ingrowth is desired, the outer surface is preferably provided with a porous coating. For example, the shell may be at least partially covered with a deposit of aluminium oxide or silicon carbide, effected by any suitable means such as a plasma torch, or a porous metal coating may be deposited electrolytically. Preferably, however, the outer shell is provided with a coating of metal beads having a diameter of from 425 mu to 850 mu, as more fully described in Bobyn, J., Pilliar, R., Cameron, H., and Weatherly, C., Clin. Orthop. and Rel. Res., No.150, 263-270 (1980). Preferably, the beads are formed from a titanium alloy (e.g. an alloy having the approximate composition Ti6A14V), and preferably are sintered onto the surface of the prosthesis in a vacuum oven at a temperature of approximately 137°C..

The invention is now further described, by way of example, with reference to the accompanying drawings, in which:

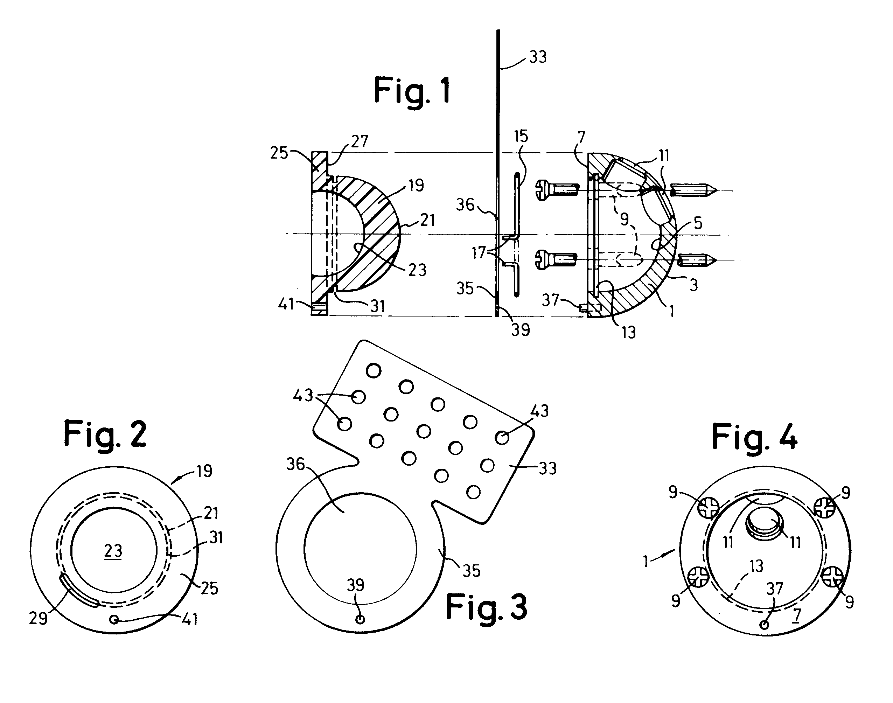

Figure 1 is an exploded sectional view through an acetabular cup according to the invention,

Figure 2 is a plan view of the lining of the device illustrated in Figure 1,

Figure 3 is a plan view of the graft support member of the device of Figure 1, and

Figure 4 is a plan view of the outer metal shell of the device of Figure 1.

Referring to the drawings, an acetabular cup comprises a generally hemispherical outer shell 1, formed from a titanium alloy. The shell has an outer hemispherical surface 3, an inner hemispherical surface 5 and a flat annular end surface 7. Screw holes 9 are formed in the end surface 7 for receiving bone screws for attachment of the shell to the acetabulum. The drawings show four such screw holes 9, but other numbers (e.g. six) may of course be used. The drawings also show two further screw holes 11, which are provided in the inner surface 5 to receive a large cancellous bone screw. Again, other numbers of such further screw holes 11 (e.g. three) may be used if desired.

The inner surface 5 also has formed therein an annular groove 13 for receiving a spring clip 15. The clip 15 is in the form of a part circle of resilient wire. The clip has an external diameter of about 38.5 mm when in the relaxed state, and an internal diameter of about 36.0 mm. The clip has upturned end portions 17, which lie substantially perpendicular to the plane of the ring, and about 10 mm apart.

The bearing surface of the cup is provided by a polyethylene lining 19 which has an outer surface 21 corresponding to the inner surface 5 of the metal shell. The lining 19 has a spherical inner surface 23 which receives the head of the femur (or the head of a femoral prosthesis, as the case may be) when the cup is in use.

The lining 19 has a rim 25 extending around the periphery thereof, rim 25 having an annular rearward surface 27. An arcuate slot 29 is formed in rim 25, to receive the end portions 17 when the cup is assembled. The lining 19 also has an annular groove 31 formed in the outer surface 21, adjacent the rim 25. The lining has a diameter of about 36.9 mm at this point, and the groove 31 is approximately 1.7 mm deep. Accordingly, the spring clip in its relaxed state is partially accommodated in groove 31, but not entirely so.

In the assembled cup, a graft support plate 33 is attached to the cup by means of an annular member 35, which is formed integrally with the plate 33, and which defines a central opening 36. The annular member 35 is sandwiched between the end surface 7 of the metal shell 1 and the rearward facing annular surface 27 of the lining 19. Rotation of the graft support plate 33 relative to the cup is prevented by means of a spigot 37 which projects from the end surface 7 of the metal shell 1, through an aperture 39 in the annular member 35, and into an aperture 41 in the lining 19. The graft support plate is provided with a large number of screw holes 43.

The procedure for use of the cup is as follows: The acetabulum is first exposed so that a good all round view of the acetabulum is obtained. The size of cup to be used is then assessed by use of a trial cup. The correct size powered hemispherical reamer is then chosen and the acetabulum reamed to the correct shape to take the cup, care being taken to ensure that the centre of rotation of the head is as near as possible to the natural centre of rotation of the hip. Using the trial cup as a guide, an appropriate hole for receiving a cancellous bone screw is drilled into the ilium, and such a screw is then used to secure the metal shell in place through screw hole 11. Depending on the bone stock available, one or more (preferably at least two) peripheral bone screws are then inserted through screw holes 9.

Assuming that a pulped bone graft is required, this is then prepared for use in the usual manner. The graft support plate 33 is then placed loosely in position by locating the aperture 39 over the spigot 37. After assessment of the extent of bone graft required, the plate is removed and then trimmed and bent to the appropriate shape The graft material is then placed in position, and the plate is relocated on the spigot 37 of the metal shell 1.

The lining 19 is then prepared for insertion in the metal shell 1 by passing the upturned ends 17 of the spring clip 15 through the elongate slot 29 in rim 25, and loosely locating the circular portion of clip 15 in the groove 31. The ends 17 are then squeezed together so that the clip 15 is received entirely within groove 31, allowing the hemispherical portion of lining 19, together with the associated clip 15, to pass through the central opening 36 of the graft support plate 33 and thus be accommodated in the metal shell 1. The ends 17 are then released, whereupon clip 15 expands to occupy groove 31. The lining 19 is thus firmly attached to the shell 1, but it is removable for revision purposes by a reversal of the above process.

Finally, the graft support plate 33 is firmly attached to the pelvis by means of a suitable number of bone screws passing through any of the screw holes 43 in the graft support plate. The large number of such screw holes affords the surgeon a wide range of suitable locations, so that maximum advantage can be taken of the existing bone stock.

If a solid bone graft is to be used, rather than a pulped graft, the procedure to be followed is generally analogous, except that the graft support plate serves a supporting role which in most cases is subsidiary to that of the screws which initially hold the graft in place.

It will be understood that the invention has been described above merely by way of example, and that many variations are possible within the scope of the invention.

**Claims**
**Claims for the following Contracting States : DE, GB, IT**

1.  An acetabular cup having a graft support plate (33) extending therefrom to support a bone graft in the vicinity of the cup, wherein the graft support plate is from 0.05 to 1.0 mm in thickness and is formed of such metal that it can readily be cut and bent to a desired shape and size.

2.  An acetabular cup according to claim 1 wherein the graft support plate (33) is from 0.1 to 0.6 mm in thickness.

3.  An acetabular cup according to claim 2 wherein the graft support plate (33) is from 0.15 to 0.4 mm in thickness.

4.  An acetabular cup according to any preceding claim wherein the graft support plate is provided with a plurality of holes (43) for receiving bone screws to attach the graft support plate to the user's pelvis.

5. An acetabular cup according to any preceding claim, said cup being in the form of a hollow generally hemispherical body (1, 19), and wherein the graft support plate (33) is attached thereto adjacent the annular end surface of the body.

6. An acetabular cup according to claim 5 wherein the cup comprises an outer generally hemispherical shell (1), and a hemispherical lining (19) which (in use of the cup) is received therein.

7. An acetabular cup according to claim 6 wherein the graft support plate (33) extends from an annular member (35) sandwiched between the annular end surface (7) of the outer shell (1) and an annular rim (25) extending around the periphery of the hemispherical lining (19).

8. An acetabular cup according to claim 7 wherein a spigot (37) is provided on the annular end surface (7) of the shell (1) and a corresponding aperture (39) is provided in the annular member (35), to prevent rotation of the graft support plate (33) relative to the outer shell (1).

9. An acetabular cup according to any of claims 6 to 8 wherein the lining (19) is secured in the outer shell (1) by means of a spring clip (15) which in the assembled cup is recieved partly in an annular groove (13) in the internal spherical surface (5) of the outer shell (1) and partly in a corresponding annular groove (31) in the outer spherical surface (21) of the lining (19).

10. An acetabular cup according to claim 9 wherein the spring clip (15) is in the form of a part-circle of suitably resilient wire, having end portions (17) which are bent out of the plane of the circle to lie generally perpendicular thereto.

11. An acetabular cup according to any of claims 6 to 10 wherein the outer shell (1) is formed from a titanium alloy.

12. An acetabular cup according to any preceding claim, having a porous coating on the outer surface thereof to allow ingrowth of bone.

**Claims for the following Contracting State : ES**

1. An acetabular cup having a graft support plate (33) extending therefrom to support a bone graft in the vicinity of the cup.

2. An acetabular cup according to claim 1, wherein the graft support plate (33) is of such thickness and of such material that it can readily be cut and bent manually to a desired shape and size.

3. An acetabular cup according to claim 2 wherein the graft support plate (33) is formed of metal and is from 0.05 to 1.0 mm in thickness.

4. An acetabular cup according to claim 3 wherein the graft support plate (33) is from 0.1 to 0.6 mm in thickness.

5. An acetabular cup according to claim 4 wherein the graft support plate (33) is from 0.15 to 0.4 mm in thickness.

6. An acetabular cup according to any preceding claim wherein the graft support plate is provided with a plurality of holes (43) for receiving bone screws to attach the graft support plate to the user's pelvis.

7. An acetabular cup according to any preceding claim, said cup being in the form of a hollow generally hemispherical body (1, 19), and wherein the graft support plate (33) is attached thereto adjacent the annular end surface of the body.

8. An acetabular cup according to claim 7 wherein the cup comprises an outer generally hemispherical shell (1), and a hemispherical lining (19) which (in use of the cup) is received therein.

9. An acetabular cup according to claim 8 wherein the graft support plate (33) extends from an annular member (35) sandwiched between the annular end surface (7) of the outer shell (1) and an annular rim (25) extending around the periphery of the hemispherical lining (19).

10. An acetabular cup according to claim 9 wherein a spigot (37) is provided on the annular end surface (7) of the shell (1) and a corresponding aperture (39) is provided in the annular member (35), to prevent rotation of the graft support plate (33) relative to the outer shell (1).

**11.** An acetabular cup according to any of claims 8 to 10 wherein the lining (19) is secured in the outer shell (1) by means of a spring clip (15) which in the assembled cup is recieved partly in an annular groove (13) in the internal spherical surface (5) of the outer shell (1) and partly in a corresponding annular groove (31) in the outer spherical surface (21) of the lining (19).

**12.** An acetabular cup according to claim 11 wherein the spring clip (15) is in the form of a part-circle of suitably resilient wire, having end portions (17) which are bent out of the plane of the circle to lie generally perpendicular thereto.

**13.** An acetabular cup according to any of claims 8 to 12 wherein the outer shell (1) is formed from a titanium alloy.

**14.** An acetabular cup according to any preceding claim, having a porous coating on the outer surface thereof to allow ingrowth of bone.

**Revendications**
**Revendications pour les Etats contractants suivants : DE, GB, IT**

**1.** Emboîture acétabulaire comprenant une plaque de support d'une greffe (33) qui en déborde et qui est destinée à supporter une greffe osseuse à proximité de l'emboîture, la plaque de support de la greffe ayant une épaisseur de 0,05 à 1,0 mm et étant réalisée en un métal tel qu'elle puisse être facilement découpée et repliée à la forme et à la dimension voulues.

**2.** Emboîture acétabulaire selon la revendication 1, dans laquelle la plaque (33) de support d'une greffe a une épaisseur de 0,1 à 0,6 mm.

**3.** Emboîture acétabulaire selon la revendication 2, dans laquelle la plaque (33) de support d'une greffe à une épaisseur de 0,15 à 0,4 mm.

**4.** Emboîture acétabulaire selon l'une quelconque des revendications précédentes, dans laquelle la plaque de support d'une greffe comporte de multiples trous (43) destinés au passage de vis à os pour fixer la plaque de support d'une greffe au bassin de l'utilisateur.

**5.** Emboîture acétabulaire selon l'une quelconque des revendications précédentes, ladite emboîture ayant la forme d'un corps creux sensiblement hémisphérique (1, 19) et la plaque (33) de support d'une greffe étant fixée à ce dernier de manière qu'elle soit contigüe à la surface annulaire extrême du corps.

**6.** Emboîture acétabulaire selon la revendication 5, dans laquelle l'emboîture se compose d'une coque extérieure sensiblement hémisphérique (1) et d'un revêtement hémisphérique (19) qui (à l'utilisation de l'emboîture) est logé dans cette dernière.

**7.** Emboîture acétabulaire selon la revendication 6, dans laquelle la plaque de support d'une greffe (33) est située dans le prolongement d'un élément annulaire (35) enserré entre la surface annulaire extrême (7) de la coque extérieure (1) et un rebord annulaire (25) situé autour de la périphérie du revêtement hémisphérique (19).

**8.** Emboîture acétabulaire selon la revendication 7, dans laquelle un ergot (37) est disposé sur la surface annulaire extrême (7) de la coque (1) et un trou correspondant (39) est réalisé dans l'élément annulaire (35) afin d'empêcher la rotation de la plaque (33) de support d'une greffe par rapport à la coque extérieure (1).

**9.** Emboîture acétabulaire selon l'une quelconque des revendications 6 à 8, dans laquelle le revêtement (19) est fixé dans la coque extérieure (1) au moyen d'un collier élastique (15) qui se loge, à l'intérieur de l'emboîture assemblée, partiellement dans une gorge annulaire (13) située dans la surface sphérique interne (5) de la coque extérieure (1) et partiellement dans une gorge annulaire correspondante (31) de la surface sphérique extérieure (21) du revêtement (9).

**10.** Emboîture acétabulaire selon la revendication 9, dans laquelle le collier élastique (15) a la forme d'un cercle partiel de fil métallique élastique convenable dont des parties extrêmes (17) sont repliées vers l'extérieur du plan du cercle de manière qu'elles soient sensiblement perpendiculaires à ce dernier.

**11.** Emboîture acétabulaire selon l'une quelconque des revendications 6 à 10, dans laquelle la coque extérieure (1) est en alliage de titane.

**12.** Emboîture acétabulaire selon l'une quelconque des revendications précédentes, comportant un enduit poreux sur sa surface extérieure afin de permettre une croissance interne d'os.

**Revendications pour l'Etat Contractant suivant : ES**

1. Emboîture acétabulaire comprenant une plaque de support d'une greffe (33) qui en déborde afin de supporter une greffe osseuse ou voisinage de l'emboîture.

2. Emboîture acétabulaire selon la revendication 1, dans laquelle la plaque (33) de support d'une greffe a une épaisseur telle et est en un matériau tel qu'elle puisse être facilement découpée et repliée à la main à la forme et à la dimension voulues.

3. Emboîture acétabulaire selon la revendication 2, dans laquelle la plaque (33) de support d'une greffe est réalisée en métal et son épaisseur est de 0,05 à 1,0 mm.

4. Emboîture acétabulaire selon le revendication 3, dans laquelle la plaque (33) de support d'une greffe a une épaisseur de 0,1 à 0,6 mm.

5. Emboîture acétabulaire selon la revendication 4, dans laquelle la plaque (33) de support d'une greffe a une épaisseur de 0,15 à 0,4 mm.

6. Emboîture acétabulaire selon l'une quelconque des revendications précédentes, dans laquelle la plaque de support d'une greffe comporte de multiples trous (43) de passage de vis à os pour la fixation de la plaque de support d'une greffe ou bassin de l'utilisateur.

7. Emboîture acétabulaire selon l'une quelconque des revendications précédentes, ladite emboîture ayant la forme d'un corps creux sensiblement hémisphérique (1, 19) et la plaque (33) de support d'une greffe lut étant fixée de manière qu'elle soit contigüe à la surface annulaire extrême du corps.

8. Emboîture acétabulaire selon la revendication 7, dans laquelle l'emboîture se compose d'une coque extérieure (1) sensiblement hémisphérique et d'un revêtement hémisphérique (19) qui (à l'utilisation de l'emboîture) est logée dans cette dernière

9. Emboîture acétabulaire selon la revendication 8, dans laquelle la plaque (33) de support d'une greffe est située dans le prolongement d'un élément annulaire (35) enserré entre la surface extrême annulaire (7) de la coque extérieure (1) et un rebord annulaire (25) situé autour de la périphérie du revêtement hémisphérique (19).

10. Emboîture acétabulaire selon la revendication 9, dans laquelle un ergot (37) est disposé sur la surface extrême annulaire (7) de la coque (1) et un trou correspondant (39) est réalisé dans l'élément annulaire (35) afin d'empêcher la rotation de la plaque (33) de support d'une greffe par rapport à la coque extérieure (1).

11. Emboîture acétabulaire selon l'une quelconque des revendications 8 à 10, dans laquelle le revêtement (19) est fixé à la coque extérieure (1) au moyen d'un collier élastique (15) qui est logé, à l'intérieur de l'emboîture assemblée, partiellement dans une gorge annulaire (13) de le surface sphérique interne (5) de le coque extérieure (1) et partiellement dans une gorge annulaire correspondante (31) de le surface sphérique extérieure (21) du revêtement (19).

12. Emboîture acétabulaire selon la revendication 11, dans laquelle le collier élastique (15) a la forme d'un cercle partiel de fil métallique élastique convenable dont des parties extrêmes (17) sont repliées vers l'extérieur du plan du cercle de manière à être sensiblement perpendiculaires à ce dernier.

13. Emboîture acétabulaire selon l'une quelconque des revendications 8 à 12, dans laquelle le coque extérieure (1) est en alliage de titane.

14. Emboîture acétabulaire selon l'une quelconque des revendications précédentes, comportant un enduit poreux à sa surface extérieure afin de permettre une croissance interne d'os.

**Patentansprüche**
**Patentansprüche für folgende Vertragsstaaten : DE, GB, IT**

1. Hüftgelenkpfanne mit einer sich davon weg erstreckenden Transplantat-Trägerplatte (33) zur Unterstützung eines Knochentransplantats in der Nähe der Pfanne, in der die Transplantat-Trägerplatte eine Dicke von 0,05 bis 1,0 mm hat und aus einem solchen Metall geformt ist, das sie leicht zu einer gewünschten Form und Größe geschnitten und gebogen werden kann.

2. Hüftgelenkpfanne nach Anspruch 1, bei der die Transplantat-Trägerplatte (33) eine Dicke von 0,1 bis 0,6 mm hat.

3. Hüftgelenkpfanne nach Anspruch 2, bei der die Transplantat-Trägerplatte (33) eine Dicke von 0,15 bis 0,4 mm hat.

4. Hüftgelenkpfanne nach einem der vorstehenden Ansprüche, bei der die Transplantat-Trägerplatte eine Vielzahl von Löchern (43) aufweist, die die Knochen-Schrauben zur Befestigung der Transplantat-Trägerplatte an dem Becken des Benutzers aufnehmen.

5. Hüftgelenkpfanne nach einem der vorstehenden Ansprüche, bei der die genannte Pfanne die Form eines hohlen, im allgemeinen halb sphärischen Körpers (1, 19) hat und bei der die Transplantat-Trägerplatte (33) daran in der Nähe der ringförmigen Endfläche des Körpers befestigt ist.

6. Hüftgelenkpfanne nach Anspruch 5, bei der die Pfanne eine äußere, im allgemeinen halb sphärische Schale (1) und eine halb sphärische Auskleidung (19) aufweist, die (bei Verwendung der Pfanne) darin aufgenommen wird.

7. Hüftgelenkpfanne nach Anspruch 6, bei der sich die Transplantat-Trägerplatte (33) von einem ringförmigen Teil (35) weg erstreckt, der zwischen der ringförmigen Endfläche (7) der äußeren Schale (1) und einem ringförmigen Rand (25) sandwichartig angeordnet ist, der sich um die Peripherie der halb sphärischen Auskleidung (19) erstreckt.

8. Hüftgelenkpfanne nach Anspruch 7, bei der ein Zapfen (37) auf der ringförmigen Endfläche (7) der Schale (1) vorgesehen ist und sich eine entsprechende Öffnung (39) im ringförmigen Teil (35) befindet, um eine Verdrehung der Transplantat-Trägerplatte (33) in bezug auf die äußere Schale (1) zu verhindern.

9. Hüftgelenkpfanne nach einem der Ansprüche 6 bis 8, bei der die Auskleidung (19) in der äußeren Schale (1) mit einer federnden Klemme (15) befestigt ist, die in der zusammengesetzten Pfanne zum Teil von einer Ringnut (13) in der inneren sphärischen Fläche (5) der äußeren Schale (1) und zum Teil von einer entsprechenden Ringnut (31) in der äußeren sphärischen Fläche (21) der Auskleidung (19) aufgenommen wird.

10. Hüftgelenkpfanne nach Anspruch 9, bei der die federnde Klemme (15) die Form eines Teilkreises aus geeignetem Federdraht mit Endteilen (17) aufweist, die aus der Ebene des Kreises herausgebogen sind, um im allgemeinen senkrecht dazu zu liegen.

11. Hüftgelenkpfanne nach einem der Ansprüche 6 bis 10, bei der die äußere Schale (1) aus einer Titan-Legierung geformt ist.

12. Hüftgelenkpfanne nach einem der vorstehenden Ansprüche, die eine poröse Beschichtung an der äußeren Oberfläche aufweist, um das Hineinwachsen des Knochens zu ermöglichen.

**Patentansprüche für folgenden Vertragsstaat : ES**

1. Hüftgelenkpfanne mit einer sich davon weg erstreckenden Transplantat-Trägerplatte (33) zur Unterstützung eines Knochentransplantats in der Nähe der Pfanne.

2. Hüftgelenkpfanne nach Anspruch 1, bei der die Transplantat-Trägerplatte (33) eine solche Dikke hat und aus einem solchen Material besteht, das sie leicht manuell zu einer gewünschten Form und Größe geschnitten und gebogen werden kann.

3. Hüftgelenkpfanne nach Anspruch 2, bei der die Transplantat-Trägerplatte (33) aus Metall geformt ist und eine Dicke von 0,05 bis 1,0 mm hat.

4. Hüftgelenkpfanne nach Anspruch 3, bei der die Transplantat-Trägerplatte (33) eine Dicke von 0,1 bis 0,6 mm hat.

5. Hüftgelenkpfanne nach Anspruch 4, bei der die Transplantat-Trägerplatte (33) eine Dicke von 0,15 bis 0,4 mm hat.

6. Hüftgelenkpfanne nach einem der vorstehenden Ansprüche, bei der die Transplantat-Trägerplatte eine Vielzahl von Löchern (43) aufweist, die die Knochen-Schrauben zur Befestigung der Transplantat-Trägerplatte an dem Becken des Benutzers aufnehmen.

7. Hüftgelenkpfanne nach einem der vorstehenden Ansprüche, bei der die genannte Pfanne die Form eines hohlen, im allgemeinen halb spärischen Körpers (1, 19) hat und bei der die Transplantat-Trägerplatte (33) daran in der Nähe der ringförmigen Endfläche des Körpers befestigt ist.

8. Hüftgelenkpfanne nach Anspruch 7, bei der die Pfanne eine äußere, im allgemeinen halb sphärische Schale (1) und eine halb sphärische Auskleidung (19) aufweist, die (bei Verwendung der Pfanne) darin aufgenommen wird.

9. Hüftgelenkpfanne nach Anspruch 8, bei der sich die Transplantat-Trägerplatte (33) von einem ringförmigen Teil (35) weg erstreckt, der zwischen der ringförmigen Endfläche (7) der äußeren Schale (1) und einem ringförmigen Rand (25) sandwichartig angeordnet ist, der sich um die Peripherie der halb sphärischen Auskleidung (19) erstreckt.

10. Hüftgelenkpfanne nach Anspruch 9, bei der ein Zapfen (37) auf der ringförmigen Endfläche (7) der Schale (1) vorgesehen ist und sich eine entsprechende Öffnung (39) im ringförmigen Teil (35) befindet, um das Verdrehen der Transplantat-Trägerplatte (33) in bezug auf die äußere Schale (1) zu verhindern.

11. Hüftgelenkpfanne nach einem der Ansprüche 8 bis 10, bei der die Auskleidung (19) an der äußeren Schale (1) mit einer federnden Klemme (15) befestigt ist, die in der zusammengesetzten Pfanne zum Teil von einer Ringnut (13) in der inneren sphärischen Fläche (5) der äußeren Schale (1) und zum Teil von einer entsprechenden Ringnut (31) in der äußeren sphärischen Fläche (21) der Auskleidung (19) aufgenommen wird.

12. Hüftgelenkpfanne nach Anspruch 11, bei der die federnde Klemme (15) die Form eines Teilkreises aus geeignetem Federdraht mit Endteilen (17) aufweist, die aus der Ebene des Kreises herausgebogen sind, um im allgemeinen senkrecht dazu zu liegen.

13. Hüftgelenkpfanne nach einem der Ansprüche 8 bis 12, bei der die äußere Schale (1) aus einer Titan-Legierung geformt ist.

14. Hüftgelenkpfanne nach einem der vorstehenden Ansprüche, die eine poröse Beschichtung an der äußeren Oberfläche aufweist, um das Hineinwachsen des Knochens zu ermöglichen.

Fig. 1

Fig. 2

Fig. 3

Fig. 4